# EUROPEAN PATENT SPECIFICATION

(11) **EP 3 522 945 B1**
(45) Date of publication and mention of the grant of the patent: **08.12.2021**
(21) Application number: 17784910.6
(22) Date of filing: 04.10.2017
(51) Int. Cl.: A61L 31/04, A61L 31/06, A61L 31/10

(54) **LAMINARY MATERIAL FOR PREVENTING POST-SURGICAL ADHESIONS**
LAMINARMATERIAL ZUR VERHINDERUNG VON POSTCHIRURGISCHEN VERKLEBUNGEN
MATÉRIAU LAMINAIRE POUR PRÉVENIR LES ADHÉRENCES POST-CHIRURGICALES

(30) Priority: 04.10.2016 US 201662403823 P
(43) Date of publication of application: 14.08.2019
(73) Proprietor: Cedar Advanced Technology Group Ltd., 6300 Zug (CH)
(72) Inventor: FARHA, Said, 8800 Thalwil (CH); FARHA, Kamal, Pleasantville, New York 10570 (US)
(74) Representative: Schaad, Balass, Menzl & Partner AG
(86) International application number: PCT/EP2017/075150
(87) International publication number: WO 2018/065444

(56) References cited:
- WO-A1-93/21858
- WO-A1-2012/142473
- US-A1- 2008 004 714

## Description

The present invention relates to a laminary material for preventing post-surgical adhesions and for removal of body fluids occuring in particular after abdominal surgery, to a corresponding kit, and to a method for preparing such a laminary material.

Adhesions are abnormal attachments between tissues and/or organs. Adhesions are in many cases post-surgical, but may also be associated with tissue trauma, infection, ischemia, hemorrhage or over-healing.

Post-surgical adhesions are among the most common complications occurring after any type of surgery. Any trauma to a tissue is normally followed by healing, which may lead to the formation of undesirable collagenous scars connecting two separate tissues at the surgical site, provided that the distance between theses tissues is small enough. Events such as excessive bleeding, inflammation, and intimate contact between tissues substantially increase the probability of adhesion formation at the site. Incidence of adhesions ranges from 60-93% after surgical procedures.

Post-surgical adhesion formation is extremely undesirable since it can cause numerous clinical complications, such as chronic pain and functional disorders, and in many cases will require re-operation. For this reason, the prevention or minimization of post-surgical adhesion formation has been the subject of many studies. It is generally believed that adhesion formation may be influenced by either disrupting the inflammatory cascade or by a mechanical barrier between affected tissues to prevent their union.

Consequently, one approach for preventing adhesion involves isolating the damaged tissue and separating it from any adjacent tissue with a biocompatible solid, fluid or gel. Thus, a physical barrier is provided between two tissues to provide separation to prevent fibrin formation between them.

Adhesion barriers are physical films, fabrics, gels or other materials that are applied between layers of tissues at the end of a surgery before the incision site is closed. While in place, the adhesion barrier acts as a physical barrier to separate traumatized tissue surfaces so that they do not adhere to one another while the tissue surfaces heal. Once the tissue surfaces heal, which is usually between three and seven days, the barrier should dissolve and be absorbed by the body.

As an example, WO 02/17853 describes hyaluronan-based adhesion compositions. More precisely, a compressed hyaluronan- or hyaluronan derivative-based composition in the form of a film having a thickness between 0.1 and 1.5 mm is disclosed.

In this publication the term "hyaluronan" is deemed to be interchangeable with and is largely used instead of the term "hyaluronic acid". Consequently, the abbreviation HA is also used to refer to hyaluronan (and hyaluronic acid). The same terminology is also used throughout the present patent (application).

Examples of commercial products providing an adhesion barrier include:
- Seprafilm^{®} (made by Genzyme) is a clear, sticky film composed of sodium hyaluronic acid with carboxymethyl cellulose (CMC). It sticks to the tissues to which it is applied and is slowly absorbed into the body over a period of seven days. It is approved for use in certain types of pelvic or abdominal surgery.
- Interceed^{®} (made by Johnson & Johnson) is a knitted fabric composed of a modified cellulose that swells and eventually gels after being placed on the injured site, and, like Seprafilm, forms a barrier and then is slowly absorbed over a period of days. It is approved for use in pelvic surgery.
- Preclude^{®} is a thin sheet of porous ePTFE (expanded polytetrafluorethylene; also called GoreTex). It provides a non-sticky, microporous insert, which is biocompatible and non-inflammatory. However, it is non-absorbable and non-degradable, so it requires a subsequent operation to remove it. Also, it must be sutured to tissue in place. For this reason, it was not approved for adhesion prevention in the USA.
- Surgiwrap^{®} (made by MAST BioSurgery) is a clear thin sheet of a dissolvable polymer similar to the kind used in dissolvable sutures. It is made of 70:30 poly(L-lactide-co-D,L-lactide), which is a synthetic analog to natural lactic acid. Naturally resorbed through bulk slow hydrolysis into lactic acid and then carbon dioxide and water. The product is approved in the USA to "reinforce soft tissues where weakness exists".
- SupraSeal^{®} is based on DL-polylactide, trimethyl carbonate and caprolactone.
- Adep^{®} (made by Baxter): Clear solution containing 4% codedextrin in buffered isotonic electrolyte solution. One to three liters of this solution are used to suspend organs and separated them from each other. Approved by the US Food and Drug Administration (FDA) for use in laparoscopy and laparotomy.
- SprayShield^{®} (made by Covidien) is a synthetic two component product that forms a gel when applied to an organ. PEG solution made of 2 components and food coloring. Reacts immediately on tissue to form a film and remains active for 5-7 days.
- Intercoat^{®} or Oxiplex^{®} (developed by Fiziomed) is a gel made from polyethylene glycol (PEG) and sodium carboxymethyl cellulose (NaCMC) and is directly applied as a single layer coating to the raw surgical surfaces at the end of the procedure.
- Coseal^{®} (made by Baxter) is a two-component, synthetic product used to seal leakage after surgery on blood vessels. It has been sprayed or applied over areas of surgical injury to reduce adhesions both in animal models and in one human trial with 71 patients undergoing myomectomy. It is marketed in Europe not only as a sealant, but also to prevent or reduce post-surgical adhesions.

An essentially different method for preventing adhesions is described in US 5,140,016. This method consists of a preliminary coating of the tissue involved in surgery by an aqueous solution of a hydrophilic, biocompatible polymer. It is stated that such pre-treatment noticeably decreases the occurrence and severity of post-surgical adhesions.

As an alternative or additional treatment, people have also tried to prevent adhesion by means of pharmacological agents, such as fibrinolytic agents, anticoagulants, and anti-inflammatories. However, since adhesion formation mechanism shares many steps with normal physiological inflammatory process, pharmacological agents may interfere with the wound healing, delaying healing. Also, ischemia and inadequate perfusion decrease drug delivery, and the peritoneal cavity has a rapid drug absorption, thus decreasing efficacy.

The analysis of the prior art reveals that the prevention of adhesions after surgery by using physical barriers is used more often than other methods. However, the success of the method is determined by such factors as the residence time of the barrier material at the site of surgery, the permeability of the barrier to the cells and proteins normally occurring during the reparative process, and the biocompatibility of the material used as a barrier.

The last mentioned factor is of extreme importance since any tissue reaction toward the material may cause the formation of additional adhesions. Therefore, biocompatible materials capable of providing reliable and efficient reduction or elimination of post-surgical adhesions are highly desirable.

While advancement in surgical techniques and equipment have reduced the incidence of adhesions in laparotomy and laparoscopic surgeries, the incidence is still high, thus requiring measures to prevent adhesions. In this context it should be noted that, although it is technically possible to apply either Seprafilm^{®} or Interceed^{®} laparoscopically, neither product is approved for this use in the USA.

A further drawback of Seprafilm^{®} is that it loses its efficacy in physiologic environment. Is also brittle and can fragment, so may be difficult to apply in certain situations.

WO9321858 discloses laminates for preventing post-operative tissue adhesion, comprising a composite of a first and a second biodegradable or bioerodable polymer layer.

US2008004714 discloses implantable anti-adhesion membranes with a first and a second layer, with a pocket edge defining a fluid-filled membrane pocket. The membrane pocket can segmented by attachments between the first and second layer.

It is therefore an objective of the present invention to provide an effective means for preventing post-surgical adhesion, which
- is biocompatible, thereby reducing the risk of inducing inflammation and adhesion formation;
- is biodegradable and/or biodegradable and easily excreted;can provide a physical barrier while simultaneously allowing the release of pharmacological agents;
- allows for physiologic cell growth;
- adheres to target surfaces without suturing;
- is flexible and easy to use even in delicate sites; and
- is fully FDA-approved, also for use in laparoscopy and laparotomy.

This object is solved by the laminary material according to claim 1, the kit according to claim 11, and the method according to claim 14. Preferred embodiments are subject of the dependent claims.

In a first aspect, the present invention refers to a laminary material for preventing post-surgical adhesions and for removal of body fluids occuring in particular after abdominal surgery.

The laminary material of the present invention comprises an adherence layer for adherence to a desired site and an at least essentially parallel spacing layer attached to the adherence layer at a plurality of attachment points. The adherence layer comprises a first polymeric film made of a first biodegradable polymer and an adherence coating, the first polymeric film having an outer surface facing away from the spacing layer and an inner surface facing towards the spacing layer. The first and second polymeric films may be in the form of a thin film, sheet or foam. At least part of the outer surface of the first polymeric film is equipped with the adherence coating. The adherence coating may be applied to the first polymeric film by coating, lamination or any other suitable process. The spacing layer comprises a second polymeric film made of a second biodegradable polymer, the second polymeric film having an outer surface facing away from the adherence layer and an inner surface facing towards the adherence layer. The adherence layer and the spacing layer define a cavity between said two layers, the cavity consisting of a plurality of interconnected compartments, which are defined by the attachment points.

In the context of the present invention, the term "attachment point" refers to a position or an area where the adherence layer and the spacing layer are attached to one another. "Attachment points" may be single points, lines or wider areas, and they may be randomly distributed over the surface of the layers or be arranged in a certain pattern, e.g. in a grid-like pattern.

The laminary material of the present invention not only provides a physical barrier, but is also suitable for delivering a pharmacologically active agent to the site of treatment. In addition, the laminary material is also able to adsorb and manage body fluids and thereby prevent leakage from the site of surgery, minimize damage and accumulate hazardous substances. Thanks to the adherence coating, the laminary material can be adhered to a tissue surface and will be kept in place, superseding a suture. Furthermore, as the polymeric films are not only biocompatible but also biodegradable, a follow-up surgery for removing the material after the desired healing time is also unnecessary. Very importantly, the laminary material is highly flexible and can be manipulated and modified (e.g. size, filling, fitting) in situ, such that it can be used in both laparoscopic and laparotomic surgeries. The laminary material of the present invention may also be used as a wound dressing or for the treatment of adhesive capsulitis of joints, such as the ankle, knee, hip or shoulder.

For instance, the first and/or second polymeric film(s) may be formed by extrusion and then laminated to one another. Alternatively, the polymeric film(s) may also be prepared by electrospinning or any other known method providing a non-woven material.

In a preferred embodiment, the first biodegradable polymer is selected from the group consisting of polylactic acid (PLA); polyglycolic acid (PGA); poly(lactic-co-glycolic acid) (PLGA); polyhydroxyalkanoate (PHA), in particular polyhydroxybutyrate (PHB); polyurethane (PU), in particular DegraBloc^{®}; cellulose, in particular carboxymethyl cellulose (CMC), ethylcellulose (e.g. Ethocel^{™} by DOW), and oxidized cellulose; polycaprolactone (PCL); hyaluronan (HA); poly (vinyl alcohol) (PVOH); polyanhydrides; and mixtures thereof. Alternatively, also gelatin, collagen, atelocollagen, albumin, alginate, cyclodextrin, chitosan, dextran, agarose, starch, polyesters, polyphosphoesters, polydioxanone (PDS), poly (amino acids), poly (imino carbonates), polyphosphates, polyphosphonates, poly cyanoacrylates, polyacetals, poly(ortho esters), polyphosphazenes, polyparadioxane or mixtures thereof may be used.

DegraBloc^{®} is a polyurethane first described by Maurer et al. ("Hepatic artery embolization with novel radiopaque polymer causes extended liver necrosis in pigs due to occlusion of the concomitant portal vein", Journal of Hepatology 2000; 32: 261-268). DegraBloc^{®} is a biocompatible, slowly degradable, radiopaque embolic agent capable of incorporating, e.g., adriamycyn (doxorubicin), a well-known anti-tumor agent. DegraBloc^{®} and similar polyurethane materials have been described in US 5,319,059 PLGA or poly(lactic-co-glycolic acid) is a copolymer which is used in a host of Food and Drug Administration (FDA) approved therapeutic devices, owing to its biodegradability and biocompatibility. PLGA is synthesized by means of ring-opening co-polymerization of two different monomers, the cyclic dimers (1,4-dioxane-2,5-diones) of glycolic acid and lactic acid. Polymers can be synthesized as either random or block copolymers thereby imparting additional polymer properties. Depending on the ratio of lactide to glycolide used for the polymerization, different forms of PLGA can be obtained: these are usually identified in regard to the molar ratio of the monomers used (e.g. PLGA 75:25 identifies a copolymer whose composition is 75% lactic acid and 25% glycolic acid). The crystallinity of PLGAs will vary from fully amorphous to fully crystalline depending on block structure and molar ratio. PLGA degrades by hydrolysis of its ester linkages in the presence of water. It has been shown that the time required for degradation of PLGA is related to the monomers' ratio used in production: the higher the content of glycolide units, the lower the time required for degradation as compared to predominantly lactide materials. An exception to this rule is the copolymer with 50:50 monomers' ratio which exhibits the faster degradation (about two months). In addition, polymers that are end-capped with esters (as opposed to the free carboxylic acid) demonstrate longer degradation half-lives. It is therefore possible to use PLGA for both the adherence layer and the spacing layer and to incorporate different degradation rates, if desired, by using different molar ratios of the monomers. For instance, it can be advantageous if the adherence layer is degraded faster than the spacing layer, or vice versa.

In a preferred embodiment, the second biodegradable polymer is selected from the group consisting of polylactic acid (PLA); polyglycolic acid (PGA); poly(lactic-co-glycolic acid) (PLGA); polyhydroxyalkanoate (PHA), in particular polyhydroxybutyrate (PHB); polyurethane (PU), in particular DegraBloc^{®}; cellulose, in particular carboxymethyl cellulose (CMC), ethylcellulose (e.g. Ethocel^{™} by DOW), and oxidized cellulose; polycaprolactone (PCL); hyaluronan (HA); poly (vinyl alcohol) (PVOH); polyanhydrides; and mixtures thereof. Alternatively, also gelatin, collagen, atelocollagen, albumin, alginate, cyclodextrin, chitosan, dextran, agarose, starch, polyesters, polyphosphoesters, polydioxanone (PDS), poly (amino acids), poly (imino carbonates), polyphosphates, polyphosphonates, poly cyanoacrylates, polyacetals, poly(ortho esters), polyphosphazenes, polyparadioxane or mixtures thereof may be used.

The first and the second biodegradable polymers may in general be selected independently from one another. Preferably, the first biodegradable polymer and the second biodegradable polymer are the same.

The above preferred polymers are not only biocompatible and biodegradable, but they are also commercially available and fully FDA approved. Furthermore, they allow for an easy and straight-forward preparation of the laminary material in any desired size and shape, and the laminary material resulting therefrom is both highly flexible and very stable.

In a preferred embodiment, the first biodegradable polymer, the second biodegradable polymer or both the first and second biodegradable polymer is/are thermo-gelling. More preferably, one or both is/are liquid at room temperature and form(s) a gel at body temperature or slightly below. Such thermo-gelling properties have been described in literature, e.g. for a biodegradable block copolymer including PLGA-PEG-PLGA (C. Wang et al., "AK12/AK19 PLGA-PEG-PLGA Triblock Polymer: Thermo-Gelation Properties and in vivo Biocompatibility", Jacobs Journal of Molecular and Translational Medicine January 2016 volume 2 Issue 1).

In a preferred embodiment, the degradation of the laminary material of the present invention may be enhanced by the addition of a salt solution or other pharmaceutically acceptable agent. This allows for a targeted degradation of the laminary material at a specific point in time, for instance by addition of said agent via a catheter or a tube and subsequent removal of the degraded material via the same catheter or tube. This is particularly advantageous in case any harmful substances have been adsorbed by the laminary material, as they can be removed at the same time.

The laminary material of the present invention may comprise only the first and the second biodegradable polymer, or there may alternatively also be additional layers of biodegradable polymer. In particular, if there are more than two layers, they may incorporate different functionalities, such as different degradation rates.

In a preferred embodiment, the adherence coating is hyaluronan (HA) or hyaluronan-based. Hyaluronan is not only FDA approved, but also guarantees a strong adherence to the tissue surface it is applied to. Furthermore, hyaluronan is exceedingly lubricious and very hydrophilic. A hyaluronan-based coating may comprise a mixture of hyaluronan with one or more other suitable polymers, but also one or more co-polymers of hyaluronan, e.g. a copolymer with a polyurethane, such as DegraBloc^{®}, or with CMC. Hyaluronan-based polymers may be fully or partially or semi-cross-linked. This provides a very flexible and at the same time very stable polymer. Alternatively, it is also possible to use cellulose.

In a preferred embodiment, the adherence layer is at least essentially flat. This allows for an optimal adherence to a tissue surface. Also, a flexible adherence layer can be fully adapted to the shape of the tissue surface.

In a first preferred embodiment, the adherence layer and the spacing layer define a single cavity between them. This cavity consists of a plurality of interconnected smaller compartments. The plurality of compartments allows for a slow release of any material contained therein to the laminary material's surroundings. Furthermore, thanks to the interconnection, any material filled into the cavity may migrate from one compartment to another and, in particular, there is a steady equalization between the different compartments, such that the thickness of the laminary material will be even over its entire extent. Therefore, if material is released from one compartment, this will be levelled off by material from the other compartments and the thickness of the laminary material will decrease evenly over the entire surface.

Alternatively, in a second preferred embodiment, the adherence layer and the spacing layer define a plurality of cavities between them. Each cavity consists of a plurality of interconnected smaller compartments, having the same advantages as described above. In addition, thanks to the presence of more than one cavity, it is possible to fill them with different materials. For instance, one cavity may contain an active agent as described below and another cavity may contain a material having adsorbing properties. By providing different types of fillings, it is possible to define different areas within the laminary material exhibiting different properties, as desired.

In a preferred embodiment, each compartment of the cavity has a size of 0.1 mm² to 20 cm², more preferably of 1 mm² to 1 cm². Within the laminary material of the present invention, all compartments may be in the same size range, or their sizes may vary. Also, there may be certain areas of the laminary material having larger compartments and others having smaller compartments.

In a preferred embodiment, the cavity contains a filler and/or one or more active agents.

Providing the cavity with a filler provides a pillow- or bubble foil-like structure, thereby defining a certain thickness of the laminary material and, thus, a certain distance between the tissues to be separated. Also, upon degradation or release of the filler, the thickness of the laminary material will decrease over time, thereby allowing a slow approaching of the surrounding tissues. Preferably, the cavity is not completely filled in order to avoid bursting.

The filler is preferably a liquid, a gel or a foam. More preferably, the filler is a liquid or a high flowing gel. For instance, a PEG hydrogel or a cellulose-based foam may be used as a filler. A foam may be prepared in supercritical CO₂, for example. Alternatively, a foam may for example also be prepared by electrospinning. The filler may also be a fully or partially hydrated hydrogel. Hydrogels may be PEG-, cellulose-, DNA- or protein-based, or it is also possible to use hybrid hydrogels. It is also possible to have several types of fillers combined in one cavity, or to provide several cavities each comprising one type of filler.

In a particularly preferred embodiment, the filler comprises one or more in situ forming polymeric formulation that is in sol form before administration and undergoes in situ gelation once administered to a patient's body. Examples of suitable thermosensitive polymers are poly-(N-isopropyl acrylamide) and poloxamers. Poloxamers - also called pluronics - are nonionic triblock copolymers composed of a central hydrophobic chain of polyoxypropylene flanked by two hydrophilic chains of polyoxyethylene. Alternatively, it is also possible to include a pH-responsive polymer, such as poly(acrylic acid) or poly(methacrylic acid).

Providing the cavity with an active agent allows for a treatment directly at the site of surgery and for a slow release over an extended period of time. The active agents may provide an additional anti-adhesion effect and/or be beneficial for the overall healing process. Also, in the case of e.g. cancer surgery, it is possible to include anti-cancer agents or other drugs. This may be beneficial at a glioblastoma resection site, pancreatic or liver resection site, or rectal cancer resection site, for instance. Thus, the laminary material according to the present invention is a delivery system which allows to deposit a high - even toxic - concentration of active agents such as anti-cancer agents or other drugs. Due to the presence of the laminary material at the site to be treated the direct interaction with for example the tumor can be forced while minimizing the circulation of said active agents in the blood.

An active agent may be supplied alone or in combination with a delivery system, in particular a targeted delivery system. For instance, an anti-tumor agent such as adriamycin may be encapsulated in liposomes, linear polymers or dendrimers or even in lipid material. Alternatively, also pH sensitive compounds or nanobased materials may be used. Examples of suitable delivery systems are described in Perche et al.: "Recent Trends in Multifunctional Liposomal Nanocarriers for Enhanced Tumor Targeting" (Journal of Drug Delivery, Volume 2013, Article ID 705265). It is also possible to include dried hydrogel particles impregnated with an antibiotic, for instance.

The active agent(s) is/are preferably selected from the group consisting of fibrinolytic agents; anticoagulants; anti-inflammatory agents, such as steroids or NSAIDs (nonsteroidal anti-inflammatory drugs); antibiotics, such as gentamycin or streptomycin; analgesics, such as morphine; anesthetics, such as novocaine or lidocaine; growth factors, such as rh-gcsf, fgf or pdgf; chemotherapeutic agents, such as adriamycin; weak acids, such as ascorbic acid or polylactic acid; polysaccharides, such as glycosaminoglycans (in particular heparin) or dextrans; proteins, such as collagen or elastin; antineoplastic agents, such as carmustine, 5-fluorouracil or vinblastine; and mixtures thereof.

Heparin is a known antithrombotic and antigrowth agent. The inclusion of such an agent in the adhesion preventing laminary material of the present invention will enhance its action by inhibiting the formation of fibrin and slowing down the migration of leucocytes.

Antineoplastic agents can be incorporated for a similar purpose. For example, when performing cancer surgery, the laminary material functions both to prevent post-surgical adhesions and also to deliver the antineoplastic agent directly to the site of the cancer where it is likely to be more effective that if it were introduced to the body intravenously. The laminary material of the present invention may also be used for the treatment of cerebral tumors.

Polylactic acid has been shown to slow down or retard the dissolution of the laminary material, meaning the residence time is increase so as to render its anti-adhesion properties more efficacious.

Antibiotics can be incorporated to prevent infection at the surgical site, and anti-inflammatory agents to prevent inflammation. A possible field of application is implant surgery, e.g. for a pacemaker.

Chemotherapeutic agents are preferably combined with mono- or polysaccharides (sugars), sugar containing natural products or honey to enhance their delivery to the tumor cells. Also the combination with other agents known to promote binding to or uptake by a tumor cell is preferred.

In a preferred embodiment, the laminary material of the present invention further comprises a contrast medium, for instance a radio-opaque substance, which allows for visualization of the laminary material within the body. Said contrast medium may be included in either one of the layers or contained within the cavity.

In a preferred embodiment, the cavity further contains a solubilizing agent forming a micellar solution. This is particularly preferable if the cavity contains one or more active agents.

In a preferred embodiment, the spacing layer and/or the adherence layer is/are perforated. Preferably, both the spacing layer and the adherence layer are perforated. The perforations preferably have a diameter of 0.5 to 2.5 µm, more preferably of 1.0 to 1.5 µm. Alternatively or in addition, it is also possible to obtain holes by electrospinning and online perforating. This will afford micro-size holes in addition to nano-size holes. Such a perforation allows for controlled release of the material contained in the cavity, in particular of a filler and/or active agent(s). Also, this provides a membrane effect.

In a preferred embodiment, the first polymeric film and/or the second polymeric film has/have a thickness of less than 100 µm, preferably a thickness of 2 to 90 µm, and more preferably of 5 to 20 µm. Such an ultrathin film can be prepared, for instance, by means of the extrusion method and die described in US 2011/0227247. This affords a highly flexible, yet very stable film. Furthermore, this provides an amorphous surface, which is better suited for degradation than a crystalline surface would be and which also has better adherence properties. The first and second polymeric film may have the same thickness or they may have different thicknesses; also, the thickness may vary within one film.

In a preferred embodiment, the outer surface of the first polymeric film and/or the outer surface of the second polymeric film is/are provided with an active coating comprising one or more active agents. This allows for site specific delivery of the active agent(s).

The active agent(s) is/are preferably selected from the group consisting of fibrinolytic agents; anticoagulants; anti-inflammatory agents, such as steroids or NSAIDs (nonsteroidal anti-inflammatory drugs); antibiotics, such as gentamycin or streptomycin; analgesics, such as morphine; anesthetics, such as novocaine or lidocaine; growth factors, such as rh-gcsf, fgf or pdgf; chemotherapeutic agents, such as adriamycin; weak acids, such as ascorbic acid or polylactic acid; polysaccharides, such as glycosaminoglycans (in particular heparin) or dextrans; proteins, such as collagen or elastin; antineoplastic agents, such as carmustine, 5-fluorouracil or vinblastine; and mixtures thereof.

Particularly preferably, the active agent(s) is/are encapsulated in microspheres. Such microspheres may be formed from, e.g. CMC, DegraBloc, HA, or mixtures or co-polymers thereof. This allows for a controlled release over a desired amount of time, while at the same time, the microspheres are held in place by the laminary material of the present invention.

In a preferred embodiment, the laminary material of the present invention further comprises Gelfoam^{®}. Gelfoam^{®} provides hemostatic properties to the laminary material of the present invention and is fully bioresorbable. It is an absorbable gelatin sponge prepared from purified pork skin gelatin USP granules and water for injection, manufactured by Pharmacia and Upjohn Company, Kalamazoo, and distributed by Pharmacia & Upjohn Co, New York. Gelfoam^{®} is preferably applied to the laminary material of the present invention in the form of a coating. Alternatively, it is also possible to insert Gelfoam^{®} into the at least one cavity.

In a preferred embodiment, the laminary material of the present invention further comprises a coating prepared by eleoctrospinning. Such a coating typically consists of fibers having a diameter in the nano- to micrometer range and up to 90% free volume with low pore size and high surface area. It can therefore absorb fluids.

Preferably, at least one compartment of the laminary material according to the present invention is connected fluid-conductive with a tube having non-perforated surface portion arranged at the outer side of the compartment and a perforated surface portion arranged inside the at least one compartment. The tube is extending from the outer side of the compartment though a tight hole of the polymeric film into the inner side of the compartment. Said tube is preferably made of a flexible material in order to increase the patient's convenience as much as possible. Preferably, it is a highly slippery material which allows a fast tube removal. The portion arranged at the outer side of the compartment is not perforated in order to ensure that the body fluids are not distributed in the body. The portion of the tube arranged inside of the compartment is perforated in order to increase the surface which can absorb the body fluids. The tube is connectable to a syringe to suck out the unwanted body fluids. After removal of the body fluids, the tube is removed from the body, whereas the laminary material preferably remains within the body to be degraded.

Preferably, at least a quarter, preferably at least half of the volume of the at least one compartment is filled with perforated surface portion of the tube. The tube may be arranged in the at least one compartment in a spiral-like manner in order to accelerate the uptake of the body fluids.

Preferably, the tube comprises a super absorbant polymer which is intended to absorb the body fluids. After absorption of the body fluids, the super absorbant polymer comprising the body fluids is either removed by a syringe or directly by pulling the tube out of the body.

A super absorbent polymer (SAP) is a type of synthetic polymeric materials capable of absorbing moisture from 500 to 1000 times its own weight. Such super absorbent polymers for medical application are known to the skilled person. Preferably, said super absorbent polymer is selected from the group consisting of polyacrylate polymers, starch graft copolymers, cellulose graft copolymers, cross-linked carboxymethylcellulose derivatives, and admixtures thereof. Further, the super absorbent polymer may additionally comprises x-ray visible particles in order to determine whether the absorption process is completed. Suitable x-ray visible particles are of metal oxides like the oxides of yttrium, ytterbium, strontium, barium, zirconium, hafnium, niobium, tantalum, tungsten, bismuth, molybdenum, tin, zinc, lanthanide elements (i.e. elements having atomic numbers ranging from 57 to 71, inclusive), cerium and combinations thereof.

Alternatively an X-ray visible compound is directly linked to the super absorbant polymer in order to make sure that the change of shape of the super absorbant polymer may be easily detected by X-ray. Alternatively, the super absorbant polymer is directly linked to DegraBloc^{®} already described above, which also allows a reliable detection by X-ray.

In a second aspect, the present invention also refers to a kit for preventing post-surgical adhesions and for removal of body fluids occuring in particular after abdominal surgery.

Said kit comprises a laminary material and an adherence coating, the laminary material having an adherence layer for adherence to a desired site and an at least essentially parallel spacing layer attached to the adherence layer at a plurality of attachment points. The adherence layer comprises a first polymeric film made of a first biodegradable polymer, the first polymeric film having an outer surface facing away from the spacing layer and an inner surface facing towards the spacing layer. The spacing layer comprises a second polymeric film made of a second biodegradable polymer, the second polymeric film having an outer surface facing away from the adherence layer and an inner surface facing towards the adherence layer. The adherence layer and the spacing layer define a cavity between said two layers, the cavity consisting of a plurality of interconnected compartments, which are defined by the attachment points.

In the kit of the present invention, the adherence coating is supplied separately or coated to at least part of the outer surface of the first polymeric film.

The kit of the present invention allows for the preparation of a laminary material for preventing post-surgical adhesions having the advantages described above. In particular, said laminary material may be prepared directly before application to the desired surgical site or in situ.

In a preferred embodiment, the kit further comprises a filler. Said filler is preferably a liquid, a gel or a foam, in particular a liquid or a high flowing gel. It is also possible to have several types of fillers combined in one cavity, or to provide several cavities each comprising one type of filler, which may or may not be the same as in other cavities. In a particularly preferred embodiment, the filler comprises one or more poloxamers.

In a preferred embodiment, the kit further comprises one or more active agents. The active agent(s) is/are preferably selected from the group consisting of fibrinolytic agents; anticoagulants; anti-inflammatory agents, such as steroids or NSAIDs (nonsteroidal anti-inflammatory drugs); antibiotics, such as gentamycin or streptomycin; analgesics, such as morphine; anesthetics, such as novocaine or lidocaine; growth factors, such as rh-gcsf, fgf or pdgf; chemotherapeutic agents, such as adriamycin; weak acids, such as ascorbic acid or polylactic acid; polysaccharides, such as glycosaminoglycans (in particular heparin) or dextrans; proteins, such as collagen or elastin; antineoplastic agents, such as carmustine, 5-fluorouracil or vinblastine; and mixtures thereof.

Particularly preferably, the kit of the present invention comprises the laminary material of the present invention as described above. Preferred embodiments of the laminary material are also preferred embodiments of the kit.

In a third aspect, the present invention also refers to a method for the preparation the laminary material of the present invention.

According to this method, a first polymeric film is formed from a first biodegradable polymer, a second polymeric film is formed from a second biodegradable polymer, and a plurality of attachment points between the first and the second polymeric film is formed such that they define a cavity consisting of a plurality of interconnected compartments between them.

Again, preferred embodiments of the laminary material as described above are also preferred embodiments of the present method.

For instance, the first and/or second polymeric film(s) may be formed by extrusion and then laminated to one another. Alternatively, the polymeric film(s) may also be prepared by electrospinning.

For obtaining the cavity consisting of a plurality of interconnected compartments, the films may be subjected to a vacuum drum defining the size of the compartments and forming the interconnections between them.

In a preferred embodiment, at least part of an outer surface of the first polymeric film is coated with an adherence coating. This may be achieved by means of a corona, plasma and/or nano treatment of the surface. More preferably, the entire outer surface of the first polymeric film is coated with an adherence coating.

Alternatively, the adherence coating may also be applied by lamination.

In a preferred embodiment, the first and/or the second polymeric film is/are perforated. More preferably, both the first and the second polymeric film are perforated. The perforation is preferably achieved by means of track-etching, e.g. as described in "Track-etch templates designed for micro- and nanofabrication" (Nuclear Instruments and Methods in Physics Research Section B: Interactions with Materials and Atoms, Volume 208, pages 115-122) or "Use of High-Technology Track-Etched Polymer Membranes in a Wide Range of Industries (American Biotechnoloy Laboratory, August 2007, pages 24-26). The perforations preferably have a diameter of 0.5 to 2.5 µm, more preferably of 1.0 to 1.5 µm.

Alternatively or in addition, it is also possible to obtain holes by electrospinning and online perforating. This will afford micro-size holes in addition to nano-size holes.

In a preferred embodiment, the first polymeric film and/or the second polymeric film is/are prepared by extrusion. Particularly preferably, the first polymeric film and/or the second polymeric film is/are prepared by extrusion according to US 2011/0227247. This allows for the preparation of an ultrathin but very stable polymeric film. Typically, the polymeric films thus obtained have a thickness of 2 to 90 µm, more preferably of 5 to 20 µm.

In a preferred embodiment, a filler is introduced into the cavity. The filler preferably is a liquid, a gel or a foam, in particular a liquid or a high flowing gel. The filler may also be a fully or partially hydrated hydrogel. It is also possible to have several types of fillers combined in one cavity, or to provide several cavities comprising more than one type of filler. In a particularly preferred embodiment, the filler comprises one or more poloxamers.

In a preferred embodiment, one or more active agents is/are introduced into the cavity. The active agent(s) is/are preferably selected from the group consisting of fibrinolytic agents; anticoagulants; anti-inflammatory agents, such as steroids or NSAIDs (nonsteroidal anti-inflammatory drugs); antibiotics, such as gentamycin or streptomycin; analgesics, such as morphine; anesthetics, such as novocaine or lidocaine; growth factors, such as rh-gcsf, fgf or pdgf; chemotherapeutic agents, such as adriamycin; weak acids, such as ascorbic acid or polylactic acid; mono- or polysaccharides, such as glycosaminoglycans (in particular heparin), glucose or dextrans; proteins, such as collagen or elastin; antineoplastic agents, such as carmustine, 5-fluorouracil or vinblastine; and mixtures thereof. Glucose conjugation is particularly favorable for the specific targeting and treatment of cancer.

The filler/and or active agent(s) may be introduced into the cavity upon preparation of the laminary material or may be inserted after placement of the laminary material into the patient. In particular, it may be filled into the cavity by means of a syringe or a catheter.

It is further possible to perform an additional surface treatment in order to obtain a non-stick surface or to provide at least one of the surfaces with a coating, such as a lubricant or a pharmaceutically active coating. Also, depending on the application, the provision of a hydrophilic coating, e.g. hyaluronan(-based), may be advantageous.

In a preferred embodiment, the outer surface of the first polymeric film and/or the outer surface of the second polymeric film is/are provided with an active coating comprising one or more active agents. The active agent(s) is/are preferably selected from the group consisting of fibrinolytic agents; anticoagulants; anti-inflammatory agents, such as steroids or NSAIDs (nonsteroidal anti-inflammatory drugs); antibiotics, such as gentamycin or streptomycin; analgesics, such as morphine; anesthetics, such as novocaine or lidocaine; growth factors, such as rh-gcsf, fgf or pdgf; chemotherapeutic agents, such as adriamycin; weak acids, such as ascorbic acid or polylactic acid; mono- or polysaccharides, such as glycosaminoglycans (in particular heparin), glucose or dextrans; proteins, such as collagen or elastin; antineoplastic agents, such as carmustine, 5-fluorouracil or vinblastine; and mixtures thereof.

Independently, the active agent(s) is/are preferably encapsulated in microspheres or any other drug delivery system, e.g. supercritical CO₂.

In a preferred embodiment, the method further includes sterilization and packaging in a sterile package.

The laminary material of the present invention may directly be prepared in the desired shape or may be adapted to the desired shape prior to placement into a patient, e.g. by cutting a smaller piece or by adhering several pieces together.

In a fourth aspect, the present disclosure also refers to the laminary material for use in a method for preventing post-surgical adhesions and for removal of body fluids occuring in particular after abdominal surgery, wherein the laminary material according to the present invention is placed into a patient.

The laminary material may be fully prepared prior to the placement, or part of the preparation may take place in situ. In particular, the application of the adherence coating or the introduction of a filler and/or one or more active agents into the cavity may be performed in situ.

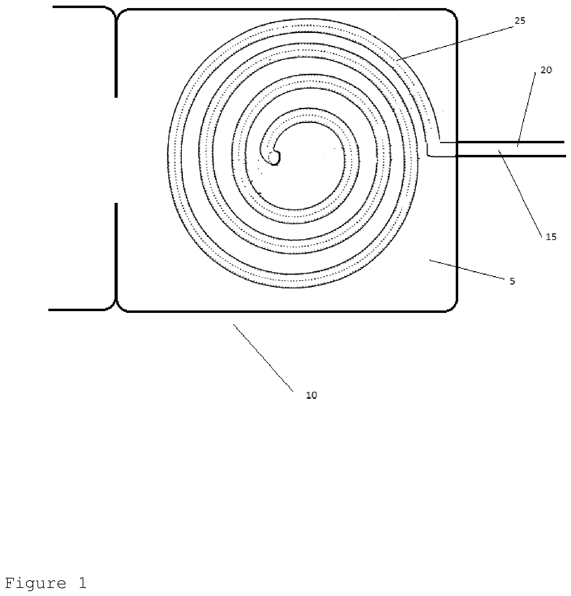

Fig. 1 shows one embodiment of the present invention.

Figure 1 shows one possible embodiment of the present invention. One compartment 5 of the laminary material 10 according to the present invention is connected fluid-conductive with a tube 15 having non-perforated surface portion 20 arranged at the outer side of the compartment and a perforated surface portion 25 arranged inside the at least one compartment.

## Claims

1. Laminary material for preventing post-surgical adhesions, comprising an adherence layer for adherence to a desired site and an at least essentially parallel spacing layer attached to the adherence layer at a plurality of attachment points, the adherence layer comprising a first polymeric film made of a first biodegradable polymer and an adherence coating, the first polymeric film having an outer surface facing away from the spacing layer and an inner surface facing towards the spacing layer, and at least part of the outer surface of the first polymeric film being coated with the adherence coating,
the spacing layer comprising a second polymeric film made of a second biodegradable polymer, the second polymeric film having an outer surface facing away from the adherence layer and an inner surface facing towards the adherence layer,
whereby the adherence layer and the spacing layer define a cavity between said two layers, the cavity consisting of a plurality of interconnected compartments, which are defined by the attachment points.

2. Laminary material according to claim 1, wherein the first biodegradable polymer and the second biodegradable polymer are independently from one another selected from the group consisting of polylactic acid; polyglycolic acid; poly(lactic-co-glycolic acid); polyhydroxyalkanoate, in particular polyhydroxybutyrate; polyurethane, in particular DegraBloc^{®}; cellulose, in particular carboxymethyl cellulose, ethylcellulose, and oxidized cellulose; polycaprolactone; hyaluronan poly (vinyl alcohol); polyanhydrides; and mixtures thereof; and wherein the first biodegradable polymer and the second biodegradable polymer are preferably the same.

3. Laminary material according to claim 1 or 2, wherein the adherence coating is hyaluronan or hyaluronan-based.

4. Laminary material according to one of claims 1 to 3, wherein the adherence layer is at least essentially flat.

5. Laminary material according to one of claims 1 to 4, wherein the adherence layer and the spacing layer define a single cavity between them.

6. Laminary material according to one of claims 1 to 5, wherein each compartment of the cavity has a size of 0.1 mm² to 20 cm², more preferably of 1 mm² to 1 cm².

7. Laminary material according to one of claims 1 to 6, wherein the cavity contains a filler and/or one or more active agents, the filler preferably being a liquid, a gel or a foam and, independently, the active agent (s) preferably being selected from the group consisting of fibrinolytic agents, anticoagulants, anti-inflammatory agents, antibiotics, analgesics, anesthetics, growth factors, chemotherapeutic agents, weak acids, mono- or polysaccharides, proteins, antineoplastic agents, and mixtures thereof.

8. Laminary material according to one of claims 1 to 7, wherein the spacing layer and/or the adherence layer is/are perforated, the perforations preferably having a diameter of 0.5 to 1.0 µm, wherein more preferably both the spacing layer and the adherence layer are perforated.

9. Laminary material according to one of claims 1 to 8, wherein the first polymeric film and/or the second polymeric film has/have a thickness of less than 100 µm, preferably a thickness of 2 to 90 µm, of 5 to 20 µm.

10. Laminary material according to one of claims 1 to 9, wherein the outer surface of the first polymeric film and/or the outer surface of the second polymeric film is/are provided with an active coating comprising one or more active agents, the active agent(s) preferably being selected from the group consisting of fibrinolytic agents, anticoagulants, anti-inflammatory agents, antibiotics, analgesics, anesthetics, growth factors, chemotherapeutic agents, weak acids, mono- or polysaccharides, proteins, antineoplastic agents, and mixtures thereof, and, independently, the active agent(s) preferably being encapsulated in microspheres.

11. Laminary material according to any of the preceding claims, wherein at least one compartment of the laminary material is connected fluid-conductive with a tube having non-perforated surface portion arranged at the outer side of the compartment and a perforated surface portion arranged inside the at least one compartment.

12. Laminary material according to claim 11, wherein the at least a quarter, preferably at least half of the volume of the at least one compartment is filled with perforated surface portion of the tube.

13. Laminary material according to claim 11 or 12, wherein the tube comprises a super absorbant polymer, optionally comprising X-ray visible particles.

14. Kit for preventing post-surgical adhesions, comprising a laminary material and an adherence coating, the laminary material having an adherence layer for adherence to a desired site and an at least essentially parallel spacing layer attached to the adherence layer at a plurality of attachment points, the adherence layer comprising a first polymeric film made of a first biodegradable polymer, the first polymeric film having an outer surface facing away from the spacing layer and an inner surface facing towards the spacing layer,
the spacing layer comprising a second polymeric film made of a second biodegradable polymer, the second polymeric film having an outer surface facing away from the adherence layer and an inner surface facing towards the adherence layer,
whereby the adherence layer and the spacing layer define a cavity between said two layers, the cavity consisting of a plurality of interconnected compartments, which are defined by the attachment points,
wherein the adherence coating is supplied separately or coated to at least part of the outer surface of the first polymeric film.

15. Kit according to claim 14, further comprising a filler and/or one or more active agents, the filler preferably being a liquid, a gel or a foam and, independently, the active agent(s) preferably being selected from the group consisting of fibrinolytic agents, anticoagulants, anti-inflammatory agents, antibiotics, analgesics, anesthetics, growth factors, chemotherapeutic agents, weak acids, mono- or polysaccharides, proteins, antineoplastic agents, and mixtures thereof.

16. Kit according to claim 14 or 15, comprising the laminary material according to one of claims 1 to 13.

17. Method for the preparation of a laminary material according to one of claims 1 to 13, wherein
a first polymeric film is formed from a first biodegradable polymer,
a second polymeric film is formed from a second biodegradable polymer, and
a plurality of attachment points between the first and the second polymeric film is formed such that they define a cavity consisting of a plurality of interconnected compartments between them.

18. Method according to claim 17, wherein at least part of an outer surface of the first polymeric film is coated with an adherence coating.

19. Method according to claim 17 or 18, wherein the first and/or the second polymeric film is/are perforated, preferably by track-etching, the perforations preferably having a diameter of 0.5 to 2.5 µm, preferably of 1.0 to 1.5 µm, wherein more preferably both the spacing layer and the adherence layer are perforated.

20. Method according to one of claims 17 to 19, wherein the first polymeric film and/or the second polymeric film is/are prepared by extrusion

21. Method according to one of claims 17 to 20, wherein a filler and/or one or more active agents is/are introduced into the cavity, the filler preferably being a liquid, a gel or a foam and, independently, the active agent(s) preferably being selected from the group consisting of fibrinolytic agents, anticoagulants, anti-inflammatory agents, antibiotics, analgesics, anesthetics, growth factors, chemotherapeutic agents, weak acids, mono- or polysaccharides, proteins, antineoplastic agents, and mixtures thereof.

22. Method according to one of claims 17 to 21, wherein the outer surface of the first polymeric film and/or the outer surface of the second polymeric film is/are provided with an active coating comprising one or more active agents, the active agent(s) preferably being selected from the group consisting of fibrinolytic agents, anticoagulants, anti-inflammatory agents, antibiotics, analgesics, anesthetics, growth factors, chemotherapeutic agents, weak acids, mono- or polysaccharides, proteins, antineoplastic agents, and mixtures thereof, and, independently, the active agent(s) preferably being encapsulated in microspheres.

## Patentansprüche

1. Schichtmaterial zum Verhindern postoperativer Adhäsionen, enthaltend eine Haftschicht zum Haften an einer gewünschten Stelle und eine zumindest im Wesentlichen parallele Abstandsschicht, die an einer Vielzahl von Befestigungspunkten an der Haftschicht befestigt ist, wobei die Haftschicht einen ersten Polymerfilm enthält, der ein erstes biologisch abbaubares Polymer und eine Haftbeschichtung enthält, wobei die erste Polymerfolie eine von der Abstandsschicht abgewandte Aussenfläche und eine der Abstandsschicht zugewandte Innenfläche aufweist und zumindest ein Teil der Aussenfläche der ersten Polymerfolie mit der Haftbeschichtung beschichtet ist,
wobei die Abstandsschicht einen zweiten Polymerfilm enthält, der aus einem zweiten biologisch abbaubaren Polymer ist, wobei der zweite Polymerfilm eine von der Haftschicht abgewandte Aussenfläche und eine der Haftschicht zugewandte Innenfläche aufweist,
wobei die Haftschicht und die Abstandsschicht einen Hohlraum zwischen den beiden Schichten definieren, wobei der Hohlraum aus mehreren miteinander verbundenen Kammern besteht, die durch die Befestigungspunkte definiert werden.

2. Schichtmaterial nach Anspruch 1, wobei das erste biologisch abbaubare Polymer und das zweite biologisch abbaubare Polymer unabhängig voneinander ausgewählt sind aus der Gruppe bestehend aus Polymilchsäure; Polyglykolsäure; Poly(Milchsäure-co-glykolsäure); Polyhydroxyalkanoat, insbesondere Polyhydroxybutyrat; Polyurethan, insbesondere DegraBloc^{®}; Cellulose, insbesondere Carboxymethylcellulose, Ethylcellulose und oxidierte Cellulose; Polycaprolacton; Hyoaluronan; Polyvinylalkohol); Polyanhydride; und Mischungen davon; und wobei das erste biologisch abbaubare Polymer und das zweite biologisch abbaubare Polymer vorzugsweise gleich sind.

3. Schichtmaterial nach Anspruch 1 oder 2, wobei die Haftbeschichtung auf Hyaluronan oder auf Hyaluronbasis basiert.

4. Schichtmaterial nach einem der Ansprüche 1 bis 3, wobei die Haftschicht zumindest im Wesentlichen flach ist.

5. Schichtmaterial nach einem der Ansprüche 1 bis 4, wobei die Haftschicht und die Abstandsschicht zwischen sich einen einzigen Hohlraum definieren.

6. Schichtmaterial nach einem der Ansprüche 1 bis 5, wobei jede Kammer des Hohlraums eine Grösse von 0,1 mm 2 bis 20 cm² hat, bevorzugter von 1 mm 2 bis 1 cm².

7. Schichtmaterial nach einem der Ansprüche 1 bis 6, wobei der Hohlraum einen Füllstoff und/oder einen oder mehrere Wirkstoffe enthält, wobei der Füllstoff vorzugsweise eine Flüssigkeit, ein Gel oder ein Schaum ist und unabhängig davon der oder die Wirkstoffe vorzugsweise ausgewählt sind aus der Gruppe bestehend aus fibrinolytischen Mitteln, Antikoagulantien, entzündungshemmenden Mitteln, Antibiotika, Analgetika, Anästhetika, Wachstumsfaktoren, Chemotherapeutika, schwachen Säuren, Mono- oder Polysacchariden, Proteinen, antineoplastischen Mitteln und Mischungen davon.

8. Schichtmaterial nach einem der Ansprüche 1 bis 7, wobei die Abstandsschicht und/oder die Haftschicht perforiert ist/sind, wobei die Perforationen vorzugsweise einen Durchmesser von 0,5 bis 1,0 µm aufweisen, wobei besonders bevorzugt sowohl die Abstandsschicht als auch die Haftschicht perforiert sind.

9. Schichtmaterial nach einem der Ansprüche 1 bis 8, wobei die erste Polymerfolie und/oder die zweite Polymerfolie eine Dicke von weniger als 100 µm, vorzugsweise eine Dicke von 2 bis 90 µm, von 5 bis 90 µm aufweisen.

10. Schichtmaterial nach einem der Ansprüche 1 bis 9, wobei die Aussenfläche des ersten Polymerfilms und/oder die Aussenfläche des zweiten Polymerfilms mit einer aktiven Beschichtung versehen ist/sind, die einen oder mehrere Wirkstoffe enthält, wobei der oder die Wirkstoff (e) vorzugsweise ausgewählt aus der Gruppe bestehend aus fibrinolytischen Mitteln, Antikoagulanzien, entzündungshemmenden Mitteln, Antibiotika, Analgetika, Anästhetika, Wachstumsfaktoren, Chemotherapeutika, schwachen Säuren, Mono- oder Polysacchariden, Proteinen, antineoplastischen Mitteln und Mischungen davon sind, und unabhängig davon der/die Wirkstoff(e) vorzugsweise in Mikrokügelchen eingekapselt ist/sind.

11. Schichtmaterial nach einem der vorhergehenden Ansprüche, wobei mindestens eine Kammer des Schichtmaterials fluidleitend mit einem Kanal verbunden ist, der einen an der Aussenseite der Kammer angeordneten nicht perforierten Oberflächenabschnitt und einen innerhalb der wenigstens einen Kammer angeordneten perforierten Oberflächenabschnitt aufweist.

12. Schichtmaterial nach Anspruch 11, wobei mindestens ein Viertel, vorzugsweise mindestens die Hälfte des Volumens der wenigstens einen Kammer mit dem Kanal mit dem perforiertem Oberflächenabschnitt gefüllt ist.

13. Schichtmaterial nach Anspruch 11 oder 12, wobei der Kanal ein superabsorbierendes Polymer enthält, das optional röntgensichtbare Partikel enthält.

14. Kit zum Verhindern postoperativer Adhäsionen, enthaltend ein Schichtmaterial und eine Haftbeschichtung, wobei das Schichtmaterial eine Haftschicht zum Haften an einer gewünschten Stelle und eine zumindest im Wesentlichen parallele Abstandsschicht aufweist, die an der Haftschicht an mehreren Befestigungspunkten befestigt ist, wobei die Haftschicht einen ersten Polymerfilm enthält, der aus einem ersten biologisch abbaubaren Polymer ist, wobei der erste Polymerfilm eine von der Abstandsschicht abgewandte Aussenfläche und eine der Abstandsschicht zugewandte Innenfläche aufweist,
wobei die Abstandsschicht einen zweiten Polymerfilm enthält, der ein zweites biologisch abbaubares Polymer enthält, wobei der zweite Polymerfilm eine von der Haftschicht abgewandte Aussenfläche und eine der Haftschicht zugewandte Innenfläche aufweist,
wobei die Haftschicht und die Abstandsschicht einen Hohlraum zwischen den beiden Schichten definieren, wobei der Hohlraum aus mehreren miteinander verbundenen Kammern besteht, die durch die Befestigungspunkte definiert werden,
wobei die Haftbeschichtung separat zugefügt oder auf mindestens einen Teil der Aussenfläche des ersten Polymerfilms aufgetragen wird.

15. Kit nach Anspruch 14, ferner enthaltend einen Füllstoff und/oder einen oder mehrere Wirkstoffe, wobei der Füllstoff vorzugsweise eine Flüssigkeit, ein Gel oder ein Schaum ist und unabhängig davon der/die Wirkstoff(e) vorzugsweise ausgewählt ist/sind aus der Gruppe bestehend aus fibrinolytisch Mittel, Antikoagulanzien, entzündungshemmende Mittel, Antibiotika, Analgetika, Anästhetika, Wachstumsfaktoren, Chemotherapeutika, schwache Säuren, Mono- oder Polysaccharide, Proteine, antineoplastische Mittel und Mischungen davon.

16. Kit nach Anspruch 14 oder 15, enthaltend das Schichtmaterial nach einem der Ansprüche 1 bis 13.

17. Verfahren zur Herstellung eines Schichtmaterials nach einem der Ansprüche 1 bis 13, wobei
ein erster Polymerfilm aus einem ersten biologisch abbaubaren Polymer gebildet wird,
ein zweiter Polymerfilm aus einem zweiten biologisch abbaubaren Polymer gebildet wird, und
eine Vielzahl von Befestigungspunkten zwischen dem ersten und dem zweiten Polymerfilm so gebildet wird, dass sie einen Hohlraum definieren, der aus einer Vielzahl von miteinander verbundenen Kammern zwischen ihnen besteht.

18. Verfahren nach Anspruch 17, wobei mindestens ein Teil einer Aussenfläche des ersten Polymerfilms mit einer Haftbeschichtung beschichtet ist.

19. Verfahren nach Anspruch 17 oder 18, wobei die erste und/oder die zweite Polymerfolie perforiert wird/werden, vorzugsweise durch Spurätzen, wobei die Perforationen vorzugsweise einen Durchmesser von 0,5 bis 2,5 µm, vorzugsweise von 1,0 bis 1,5 µm aufweisen, wobei mehr vorzugsweise sind sowohl die Abstandsschicht als auch die Haftschicht perforiert.

20. Verfahren nach einem der Ansprüche 17 bis 19, wobei die erste Polymerfolie und/oder die zweite Polymerfolie durch Extrusion hergestellt wird/werden.

21. Verfahren nach einem der Ansprüche 17 bis 20, wobei ein Füllstoff und/oder ein oder mehrere Wirkstoffe in die Kammer eingebracht werden, wobei der Füllstoff vorzugsweise eine Flüssigkeit, ein Gel oder ein Schaum ist und unabhängig davon der/die Wirkstoff(e) vorzugsweise ausgewählt ist/sind aus der Gruppe bestehend aus fibrinolytischen Mitteln, Antikoagulanzien, entzündungshemmenden Mitteln, Antibiotika, Analgetika, Anästhetika, Wachstumsfaktoren, Chemotherapeutika, schwachen Säuren, Mono- oder Polysacchariden, Proteinen, antineoplastischen Mitteln und Mischungen davon.

22. Verfahren nach einem der Ansprüche 17 bis 21, wobei die äussere Oberfläche des ersten Polymerfilms und/oder die äussere Oberfläche des zweiten Polymerfilms mit einer aktiven Beschichtung versehen wird, die einen oder mehrere Wirkstoffe enthält, wobei der/die Wirkstoff(e) vorzugsweise ausgewählt ist/sind aus der Gruppe bestehend aus fibrinolytischen Mitteln, Antikoagulanzien, entzündungshemmenden Mitteln, Antibiotika, Analgetika, Anästhetika, Wachstumsfaktoren, Chemotherapeutika, schwachen Säuren, Mono- oder Polysacchariden, Proteinen, antineoplastischen Mitteln und Mischungen davon, und unabhängig davon, der/die Wirkstoff(e) vorzugsweise in Mikrokügelchen eingekapselt ist/sind.

## Revendications

1. Matériau laminaire pour prévenir les adhérences post-chirurgicales, comprenant une couche d'adhérence destinée à adhérer à un site souhaité et une couche d'espacement au moins essentiellement parallèle fixée à la couche d'adhérence en une pluralité de points de fixation,
la couche d'adhérence comprenant un premier film polymère fait d'un premier polymère biodégradable et d'un revêtement d'adhérence, le premier film polymère ayant une surface extérieure tournée à l'opposé de la couche d'espacement et une surface intérieure tournée vers la couche d'espacement, et au moins une partie de la surface extérieure du premier film polymère étant revêtue du revêtement d'adhérence,
la couche d'espacement comprenant un second film polymère fait d'un second polymère biodégradable, le second film polymère ayant une surface externe tournée à l'opposé de la couche d'adhérence et une surface interne tournée vers la couche d'adhérence,
dans lequel la couche d'adhérence et la couche d'espacement définissent une cavité entre lesdites deux couches, la cavité consistant en une pluralité de compartiments interconnectés, qui sont définis par les points de fixation.

2. Matériau laminaire selon la revendication 1, dans lequel le premier polymère biodégradable et le second polymère biodégradable sont indépendamment l'un de l'autre choisis dans le groupe constitué par l'acide polylactique ; l'acide polyglycolique ; le poly(acide lactique-co-glycolique) ; le polyhydroxyalcanoate, en particulier le polyhydroxybutyrate ; polyuréthane, en particulier DegraBloc^{®} ; cellulose, en particulier carboxyméthylcellulose, éthylcellulose et cellulose oxydée ; polycaprolactone ; hyaluronane ; poly(alcool vinylique) ; polyanhydrides ; et leurs mélanges ; et dans lequel le premier polymère biodégradable et le second polymère biodégradable sont de préférence les mêmes.

3. Matériau laminaire selon la revendication 1 ou 2, dans lequel le revêtement d'adhérence est du hyaluronan ou à base de hyaluronan.

4. Matériau laminaire selon l'une des revendications 1 à 3, dans lequel la couche d'adhérence est au moins essentiellement plane.

5. Matériau laminaire selon l'une des revendications 1 à 4, dans lequel la couche d'adhérence et la couche d'espacement définissent une seule cavité entre elles.

6. Matériau lamellaire selon l'une des revendications 1 à 5, dans lequel chaque compartiment de la cavité a une taille de 0,1 mm² à 20 cm², plus préférablement de 1 mm² à 1 cm².

7. Matériau laminaire selon l'une des revendications 1 à 6, dans lequel la cavité contient une charge et/ou un ou plusieurs agents actifs, la charge étant de préférence un liquide, un gel ou une mousse et, indépendamment, le ou les agents actifs étant de préférence choisis dans le groupe constitué par les agents fibrinolytiques, les anticoagulants, les agents anti-inflammatoires, les antibiotiques, les analgésiques, les anesthésiques, les facteurs de croissance, les agents chimiothérapeutiques, les acides faibles, les mono- ou polysaccharides, les protéines, les agents antinéoplasiques, et leurs mélanges.

8. Matériau laminaire selon l'une des revendications 1 à 7, dans lequel la couche d'espacement et/ou la couche d'adhérence est/sont perforée(s), les perforations ayant de préférence un diamètre de 0,5 à 1,0 µm, dans lequel, plus préférablement, tant la couche d'espacement que la couche d'adhérence sont perforées.

9. Matériau lamellaire selon l'une des revendications 1 à 8, dans lequel le premier film polymérique et/ou le second film polymérique a/ont une épaisseur inférieure à 100 µm, de préférence une épaisseur de 2 à 90 µm, de 5 à 20 µm.

10. Matériau laminaire selon l'une des revendications 1 à 9, dans lequel la surface extérieure du premier film polymère et/ou la surface extérieure du second film polymère est/sont pourvue(s) d'un revêtement actif comprenant un ou plusieurs agents actifs, le ou les agents actifs étant de préférence choisis dans le groupe constitué par les agents fibrinolytiques, les anticoagulants, les agents anti-inflammatoires, les antibiotiques, les analgésiques, les anesthésiques, les facteurs de croissance, les agents chimiothérapeutiques, les acides faibles, les mono- ou polysaccharides, les protéines, les agents antinéoplasiques et leurs mélanges, et, indépendamment, le ou les agents actifs étant de préférence encapsulés dans des microsphères.

11. Matériau laminaire selon l'une quelconque des revendications précédentes, dans lequel au moins un compartiment du matériau laminaire est relié par conduction de fluide à un tube ayant une partie de surface non perforée disposée sur le côté extérieur du compartiment et une partie de surface perforée disposée à l'intérieur du au moins un compartiment.

12. Matériau laminaire selon la revendication 11, dans lequel au moins un quart, de préférence au moins la moitié du volume du au moins un compartiment est rempli de la partie de surface perforée du tube.

13. Matériau laminaire selon la revendication 11 ou 12, dans lequel le tube comprend un polymère super absorbant, comprenant éventuellement des particules visibles aux rayons X.

14. Kit pour la prévention des adhérences post-chirurgicales, comprenant un matériau laminaire et un revêtement d'adhérence, le matériau laminaire ayant une couche d'adhérence pour l'adhérence à un site souhaité et une couche d'espacement au moins essentiellement parallèle fixée à la couche d'adhérence en une pluralité de points de fixation, la couche d'adhérence comprenant un premier film polymère fait d'un premier polymère biodégradable, le premier film polymère ayant une surface extérieure tournée à l'opposé de la couche d'espacement et une surface intérieure tournée vers la couche d'espacement, la couche d'espacement comprenant un second film polymère fait d'un second polymère biodégradable, le second film polymère ayant une surface externe tournée à l'opposé de la couche d'adhérence et une surface interne tournée vers la couche d'adhérence, dans lequel la couche d'adhérence et la couche d'espacement définissent une cavité entre lesdites deux couches, la cavité consistant en une pluralité de compartiments interconnectés, qui sont définis par les points de fixation, dans lequel la couche d'adhérence est fournie séparément ou appliquée sur au moins une partie de la surface extérieure du premier film polymère.

15. Kit selon la revendication 14, comprenant en outre une charge et/ou un ou plusieurs agents actifs, la charge étant de préférence un liquide, un gel ou une mousse et, indépendamment, le ou les agents actifs étant de préférence choisis dans le groupe constitué par les agents fibrinolytiques, les anticoagulants, les agents anti-inflammatoires, les antibiotiques, les analgésiques, les anesthésiques, les facteurs de croissance, les agents chimiothérapeutiques, les acides faibles, les mono- ou polysaccharides, les protéines, les agents antinéoplasiques et leurs mélanges.

16. Kit selon la revendication 14 ou 15, comprenant le matériau laminaire selon l'une des revendications 1 à 13.

17. Procédé de préparation d'un matériau laminaire selon l'une des revendications 1 à 13, dans lequel un premier film polymère est formé à partir d'un premier polymère biodégradable, un second film polymère est formé d'un second polymère biodégradable, et une pluralité de points de fixation entre le premier et le second film polymère est formée de telle sorte qu'ils définissent une cavité constituée d'une pluralité de compartiments interconnectés entre eux.

18. Procédé selon la revendication 17, dans lequel au moins une partie d'une surface extérieure du premier film polymère est revêtue d'un revêtement d'adhérence.

19. Procédé selon la revendication 17 ou 18, dans lequel le premier et/ou le second film polymère est/sont perforé(s), de préférence par track-etching, les perforations ayant de préférence un diamètre de 0,5 à 2,5 µm, de préférence de 1,0 à 1,5 µm, dans lequel, plus préférablement, la couche d'espacement et la couche d'adhérence sont toutes deux perforées.

20. Procédé selon l'une des revendications 17 à 19, dans lequel le premier film polymère et/ou le second film polymère est/sont préparé(s) par extrusion.

21. Procédé selon l'une des revendications 17 à 20, dans lequel une charge et/ou un ou plusieurs agents actifs est/sont introduits dans la cavité, la charge étant de préférence un liquide, un gel ou une mousse et, indépendamment, le ou les agents actifs étant de préférence choisis dans le groupe constitué par les agents fibrinolytiques, les anticoagulants, les agents anti-inflammatoires, les antibiotiques, les analgésiques, les anesthésiques, les facteurs de croissance, les agents chimiothérapeutiques, les acides faibles, les mono- ou polysaccharides, les protéines, les agents antinéoplasiques, et leurs mélanges.

22. Procédé selon l'une des revendications 17 à 21, dans lequel la surface extérieure du premier film polymère et/ou la surface extérieure du second film polymère est/sont pourvue(s) d'un revêtement actif comprenant un ou plusieurs agents actifs, le ou les agents actifs étant de préférence choisis dans le groupe constitué par les agents fibrinolytiques, les anticoagulants, les agents anti-inflammatoires, les antibiotiques, les analgésiques, les anesthésiques, les facteurs de croissance, les agents chimiothérapeutiques, les acides faibles, les mono- ou polysaccharides, les protéines, les agents antinéoplasiques et leurs mélanges, et, indépendamment, le ou les agents actifs étant de préférence encapsulés dans des microsphères.
